# FASCICULE DE BREVET EUROPEEN

(11) **EP 1 644 400 B1**
(45) Date de publication et mention de la délivrance du brevet: **06.08.2008**
(21) Numéro de dépôt: 04767259.7
(22) Date de dépôt: 04.06.2004
(51) Int. Cl.: C07J 9/00, C07J 75/00

(54) **PROCEDE DE RECUPERATION DE STEROLS**
VERFAHREN ZUR RÜCKGEWINNUNG VON STEROL
STEROL RECOVERING METHOD

(30) Priorité: 10.06.2003 FR 0306926
(43) Date de publication de la demande: 12.04.2006
(73) Titulaire: Novance, 60206 Compiegne (FR)
(72) Inventeur: CHARLEMAGNE, Dominique, F-60200 Compiègne (FR); BOSTYN, Stéphane, F-45740 Lailly en Val (FR); DAGUET, David, F-17139 Dompierre sur Mer (FR); COIC, Jean-Pierre, 45370 Clery Saint Andre (FR)
(74) Mandataire: Portal, Gérard
(86) Numéro de dépôt international: PCT/FR2004/001391
(87) Numéro de publication internationale: WO 2004/111073

(56) Documents cités:
- WO-A-01/32681
- US-A- 2 835 682
- US-A- 3 840 570
- US-A- 4 374 776

## Description

La présente invention a pour objet l'utilisation d'un produit issu du traitement d'huiles de composés d'origine végétale oléagineuse, pour la récupération de stérols. Elle a également pour objet un procédé de récupération de stérols contenus dans les huiles de composés d'origine végétale oléagineuse. Le procédé est plus particulièrement un procédé de récupération à partir d'un produit issu d'un traitement dans lequel l'huile a subi une étape de transestérification. Ce procédé est tout particulièrement approprié pour la récupération de phytostérols.

Les stérols, tels que le β-sitostérol, le stigmastérol, le D7- le stigmastérol, le brassicastérol, le D5-avénastérol; le campestérol, leurs dérivés saturés comme le sitostanol et le campestanol, ainsi que les tocophérols, sont des composés bien connus et dont l'intérêt est sans cesse croissant. En effet, certains d'entre eux sont des agents favorisant la réduction du taux de cholestérol chez l'homme, ce qui en fait des additifs recherchés dans l'alimentation. Ils trouvent aussi une application dans l'industrie de la cosmétique, notamment pour leurs propriétés anti-inflammatoires et régénérantes. Par ailleurs, ils peuvent constituer des intermédiaires dans la synthèse de composés ayant une activité thérapeutique comme les stéroïdes par exemple.

On trouve des stérols dans les huiles d'origine végétale mais leur teneur ne dépasse pas 1 à 2 % dans les huiles brutes. Or à de telles teneurs et malgré l'intérêt de ces composés, il n'est pas économiquement viable de les extraire.

Par contre, lorsque l'on traite des huiles telles que de l'huile de tall pour les raffiner par distillation, on obtient des distillats de désodorisation dont la teneur en stérols est multipliée par 10 par rapport à celle dans les huiles brutes, et leur récupération devient alors envisageable.

Ainsi, on connaît la récupération de stérols obtenus après la mise en oeuvre d'un raffinage dit physique (par distillation) d'huiles, soit par extraction au moyen d'un solvant approprié, soit par saponification puis extraction au moyen d'un solvant approprié.

Dans un procédé plus récent que celui qui vient d'être mentionné ci-dessus, les stérols sont récupérés en mettant en oeuvre une étape de transestérification (alcoolyse) avec un alcool léger comme le méthanol, en présence d'un catalyseur à base d'étain, en autoclave à une température de l'ordre de 180 à 220°C. Le produit résultant est ensuite soumis à une étape de distillation sous un vide poussé (inférieur à 0,5 mbar) et à une température relativement élevée (200 à 300°C).

Le document WO 01/32681 décrit un procédé de récupération de stérols à partir d'huiles d'origine végétale, dans lequel on cristallise des stérols dans un mélange réactionnel issu de la transestérification d'huiles, pour obtenir un produit impur à base de stérols (84% en poids de stérols), on saponifie le produit impur, puis on cristallise les stérols présents dans le produit de saponification, pour obtenir des stérols de grande pureté. Ce procédé n'est toutefois pas économiquement performant car la première cristallisation, à partir du mélange réactionnel de transestérification est très longue.

Dans le document US 4,374,776, il est décrit un procédé de récupération de stérols à partir de résidus de distillation d'une huile ayant subi au préalable une étape (raffinage dit chimique) de clivage, pour l'obtention d'acide, ou de transestérification, pour l'obtention d'esters. Les résidus de distillations sont des résidus à faible teneur en stérols, moins de 15% en poids, et sont ensuite transestérifiés selon une méthode comparable à celle mentionnée auparavant, puis distillés à nouveau. Le distillat obtenu ne comprend pas plus de 50 % en poids de stérols.

L'inconvénient de ces procédés est qu'il requiert de disposer d'appareillages supplémentaires pour effectuer l'étape de transestérification et de distillation en vue de récupérer les stérols et cela représente un coût important. En outre, les stérols, surtout sous forme alcool, sont des composés sensibles à la chaleur et le fait de les soumettre à divers traitements thermiques notamment dans des conditions difficiles (vide poussé), représente un risque de voir le rendement de la séparation diminué. Force est de constater qu'aucun des moyens qui ont proposés pour récupérer les stérols ne satisfait à une exigence de rentabilité (productivité, rendement) suffisante.

D'autre part, on cherche à valoriser la filière des produits issus des huiles d'origine végétale, c'est à dire à utiliser la plus grande quantité possible de ces produits, de manière rentable, et à détruire par combustion ou mise au rebut, la plus petite quantité de produit. Dans bien des cas une telle valorisation naît d'une identification et d'une optimisation des flux de produits et de sous produits, et d'une utilisation adéquate. Autrement, les opérations ne sont pas viables économiquement, et il n'y a pas de valorisation. Une telle valorisation a des effets bénéfiques sur l'environnement, notamment, en ce qu'elle limites les mises au rebut ou les combustions de matière. Les opérations décrites ci-dessus... Aucun des traitements qui ont été proposés jusqu'à maintenant n'ont permis une valorisation optimisée.

La présente invention a pour objet une valorisation de la filière des produits issus des huiles d'origine végétale, qui ne présente pas les inconvénients mentionnés ci-dessus. Elle a également pour objet un procédé de récupération de stérols ne mettant pas en jeu d'étapes de distillation qui risquent de diminuer le rendement de récupération desdits composés.

A cet effet l'invention propose d'utiliser un produit issu du traitement d'huiles de composés d'origine végétale oléagineuse pour la récupération de stérols, caractérisé en ce que le produit utilisé est un résidu lourd issu d'une distillation d'un produit de transestérification de l'huile, ledit résidu comprenant:
- entre 15% et 50 % en poids de stérols,
- et des composés saponifiables.

L'invention propose également un procédé de récupération de stérols à partir d'un produit issu du traitement d'huiles de composés d'origine végétale oléagineuse, caractérisé en ce que:
- le produit utilisé est un résidu lourd issu d'une distillation d'un produit de transestérification de l'huile, ledit résidu comprenant entre 10% et 50 % en poids de stérols, et des composés saponifiables, et
- le procédé comprend les étapes suivantes:
   a) on saponifie les composés saponifiables par mise en contact du résidu avec une base en présence d'un solvant comprenant de l'eau et un co-solvant;
   b) on cristallise les stérols sous la forme de particules solides en présence du mélange à un rapport pondéral eau / co-solvant supérieur à 1/1, le cas échéant en ajoutant de l'eau de manière à atteindre ce rapport pondéral, de manière à obtenir une dispersion,
   c) on sépare les stérols cristallisés du mélange solvant issu de l'étape b), comprenant les composés saponifiés obtenus, par essorage de la dispersion,
   d) on lave éventuellement les stérols avec un deuxième liquide et l'on élimine ledit liquide par essorage ou centrifugation,
   e) on reproduit éventuellement au moins une fois l'étape d);
   f) on sèche et récupère les stérols.

Il est à noter que le procédé selon l'invention a priori ne pouvait être favorablement envisagé par l'homme du métier. En effet, les résidus traités selon l'invention par saponification ont pour résultat l'obtention d'un mélange dont les caractéristiques sont telles qu'il était loin d'être évident de pouvoir extraire avec un bon rendement les composés souhaités. Ainsi les méthodes classiques de séparation et de filtration présentent des inconvénients importants lors de leur mise en oeuvre. Par exemple, un faible débit de passage de la suspension à traiter au travers d'un filtre, d'une toile ou d'une membrane, dû à un colmatage rapide de l'appareil de séparation, ou encore l'utilisation d'un matériel coûteux, comme par exemple des évaporateurs en couche mince, ou des colonnes de distillation qui peuvent être de plus la cause d'une dégradation thermique des composés à séparer, etc. Aucun indice ne permettait donc à l'homme du métier d'envisager une valorisation efficace tant techniquement qu'économiquement.

Mais d'autres buts et avantages de la présente invention apparaîtront plus clairement à la lecture de la description et des exemples qui vont suivre.

Ainsi que cela a été indiqué auparavant, le produit valorisé est d'origine végétale oléagineuse. II s'agit d'une huile d'origine végétale, de préférence oléagineuse.

En tant qu'huile convenable, on peut citer notamment est l'huile de colza, l'huile de tournesol, l'huile d'arachide, l'huile d'olive, l'huile de noix, l'huile de maïs, l'huile de soja, l'huile d'avocat, l'huile de lin, l'huile de chanvre, l'huile de noix de macadamia, l'huile de pépins de raisin, l'huile de coprah, l'huile de palme, l'huile de graines de coton, l'huile de sésame, l'huile de ricin, l'huile de babassu, l'huile de palmiste, l'huile de tung, ou le beurre de karité, seule ou en mélange.

De préférence, on utilise les huiles de colza, tournesol, soja, seules ou en mélange.

Le produit utilisé selon l'invention est un résidu lourd issu d'une distillation d'un produit de transestérification de l'huile ou du mélange d'huiles.

Cette étape de transestérification est réalisée de manière classique en soi. Par exemple, l'huile ou le mélange d'huiles à traiter, est mise en contact avec au moins un alcool saturé comprenant moins de 6 atomes de carbone, de préférence le méthanol ou l'éthanol, en présence d'un catalyseur basique comme les alcoolates (méthylate de sodium par exemple) et les hydroxydes de métal alcalin, comme la soude.

Généralement cette opération est réalisée à une température comprise entre 20 et 60°C.

Le produit issu de cette réaction est ensuite décanté afin de séparer les esters de la phase aqueuse. Les esters peuvent être lavés, séparés du milieu de lavage puis distillés de manière classique, afin de récupérer, avec les produits volatiles (tête de colonne), les esters obtenus, et un résidu de distillation (pied de colonne).

Le résidu de distillation qui est valorisé selon l'invention et utilisé comme produit de départ dans le procédé selon l'invention comprend des stérols à une teneur qui est comprise entre 15 et 50 % en poids. De préférence, la teneur en stérols est comprise entre 15 et 30%, et plus préférentiellement encore entre 20 et 25 % en poids. Le résidu comprend, par ailleurs, des composés saponifiables, qui sont plus particulièrement des acides gras, des esters d'acides gras, des mono-, di- et tri- glycérides. Le résidu peut éventuellement de même comprendre des composés solubles issus de la graine à partir de laquelle l'huile est obtenue, comme par exemple des phospholipides.

La teneur en composés saponifiables est déterminée par des méthodes connues de l'homme du métier, notamment en mesurant l'indice de saponification du résidu. L'indice de saponification est mesuré selon la norme NF T60-206. L'indice de saponification du résidu est généralement compris entre 100 et 200 g de potasse par gramme de résidu, de préférence entre 100 et 150 grammes de potasse par gramme de résidu.

On détaille à présent les étapes du procédé selon l'invention.

Selon la première étape du procédé (étape a), le résidu est mis en contact avec une base en présence d'un mélange solvant dans le but de saponifier les composés saponifiables présents dans ledit résidu.

Plus particulièrement, la base est choisie parmi les hydroxydes de métal alcalin ou alcalino-terreux. De préférence, la base est la potasse ou la soude.

Par ailleurs, la quantité de base ajoutée durant cette étape a) est supérieure ou égale à la quantité nécessaire pour saponifier la totalité des composés saponifiables présents dans le réactif. La détermination de la teneur en composés saponifiables est faite selon les méthodes connues de l'homme du métier, notamment en mesurant l'indice de saponification.

De manière avantageuse, la quantité de base ajoutée est comprise entre la quantité stoechiométrique, c'est-à-dire la quantité suffisante pour que tous les composés saponifiables soient saponifiés, et un excès de 900 % par rapport à la quantité stoechiométrique, de préférence une quantité de base comprise entre la quantité stoechiométrique et un excès de 600 % et idéalement entre la quantité stoechiométrique et 100% excès. Il n'y a en effet pas d'avantage particulier à réaliser cette étape a) en présence d'un excès de base. Au contraire, il peut y avoir des inconvénients à effectuer cette étape en présence d'un grand excès de base. Ainsi, le mélange réactionnel obtenu à l'issue de cette étape peut voir sa viscosité augmenter de manière importante, ce qui compliquera la mise en oeuvre de l'étape ultérieure de séparation des stérols obtenus. Par ailleurs, dans de telles conditions, un phénomène de moussage peut être observé. Le pH élevé du milieu réactionnel en fin de saponification peut également être source de corrosion des installations si l'excès de potasse est trop important.

De préférence, l'opération est réalisée avec une quantité stoechiométrique de base.

L'étape est mise en oeuvre en présence d'un mélange solvant. Le mélange solvant comprend de l'eau et un co-solvant. II est de préférence choisi de telle sorte qu'il solubilise, à une température inférieure ou égale à 20°C, les composés saponifiés et qui ne solubilise pas les stérols.

Plus particulièrement, mélange solvant est choisi de telle sorte que la limite de solubilité des composés saponifiables dépasse 90 % en poids, plus particulièrement dépasse 95 % en poids, et de préférence est de 100% en poids, dans une gamme de température comprise entre la température à laquelle est mise en oeuvre l'étape a) et 5°C.

D'autre part, solvant est avantageusement choisi de manière à ce que la limite de solubilité des stérols présents soit inférieure à 10% en poids, de préférence inférieure à 5 % en poids dans ledit premier solvant, et cela dans la gamme de température précisée ci-dessus.

Enfin, le solvant est de préférence choisi de telle sorte qu'à la température à laquelle l'étape a) est effectuée, le mélange réactionnel est monophasique.

Le co-solvant est partiellement ou totalement soluble dans l'eau. De préférence, la teneur en co-solvant ne dépasse pas la limite de solubilité de ce dernier dans l'eau, dans les mêmes conditions de température que celles énoncées plus haut.

A titre de co-solvant convenable à la réalisation de l'invention, on peut choisir les alcools saturés comprenant 1 à 4 atomes de carbone.

A titre d'exemples de composés susceptibles d'être employés en tant que co-solvant, on peut citer le méthanol, et l'éthanol. Selon un mode de réalisation préféré, si un co-solvant est présent, il est choisi parmi les alcools, l'éthanol étant préféré.

Lors de l'étape a), le rapport pondéral eau / co-solvant du mélange solvant peut varier dans un large domaine, dès l'instant que le mélange réactionnel est monophasique. Conformément à un mode de réalisation de l'invention, le rapport pondéral eau / co-solvant est au moins de 1/25.

Selon une variante particulièrement intéressante de l'invention, le mélange solvant mis en oeuvre lors de l'étape a) est tel que le rapport pondéral eau / co-solvant est supérieur à 1/1, inférieur ou égal à 3/1. De préférence, le rapport pondéral eau / co-solvant est compris entre 1,5/1 et 3/1, encore plus préférentiellement compris entre 1,5/1 et 2,53/1.

En outre, l'étape a) est effectuée de préférence avec une quantité de solvant correspondant à un rapport pondéral premier solvant / réactif compris entre 2/1 et 10/1, de préférence entre 2/1 et 5/1.

Avantageusement, l'étape a) est réalisée en mélangeant la base avec le premier solvant. On ajoute ensuite le réactif au mélange obtenu. De préférence on opère de manière à homogénéiser le résidu dans le mélange solvant/base, pour obtenir de préférence un mélange monophasique. Une température de l'ordre de 50°C est par exemple adaptée.

L'opération a de préférence lieu sous agitation.

Quant à la température à laquelle la saponification est effectuée, elle est habituellement supérieure à 20°C. De préférence, la réaction est mise en oeuvre à la température de reflux du co-solvant, ou du mélange d'eau et de co-solvant. A température de saponification est par exemple d'environ 80°C.

L'avancement de la réaction de saponification est habituellement suivi en contrôlant l'indice de saponification du mélange réactionnel, par prélèvement régulier d'échantillons. On poursuit avantageusement la réaction jusqu'à ce que au moins 90% des composés saponifiables aient été saponifiés On considère que ce taux de 90% est atteint lorsque la valeur de l'indice de saponification atteint un plateau (valeur constante entre deux prélèvements, aux incertitudes près).

A l'issue de l'étape a), par exemple lorsque l'indice de saponification n'évolue plus de manière substantielle, on cristallise les stérols (étape b), de manière à obtenir une dispersion. Cette cristallisation est effectuée à un rapport eau / co-solvant pour le mélange solvant supérieur à 1/1. Ainsi, si le rapport pondéral eau / co-solvant du mélange solvant mis en oeuvre dans l'étape a) est inférieur ou égal à 1/1, on introduit de l'eau dans le mélange réactionnel, de telle sorte que le rapport pondéral eau / co-solvant soit supérieur à 1/1. II n'est pas exclu d'ajouter de l'eau si le rapport pondéral eau / co-solvant du mélange solvant mis en oeuvre dans l'étape a) est supérieur à 1/1. De préférence, la teneur en eau, avec l'eau éventuellement ajoutée, lors de l'étape b) est telle que le rapport pondéral eau / co-solvant est inférieur ou égal à 3/1, avantageusement, compris entre 1,5/1 et 3/1, de préférence compris entré 1,5/1 et 2,5/1.

Avantageusement, l'introduction d'eau, si elle a lieu, est effectuée à la température du milieu où la réaction de la saponification est effectuée. Il s'agit en général de la température de reflux du co-solvant ou du mélange d'eau et de co-solvant. La température est par exemple d'environ 80°C.

Cette étape est plus spécialement effectuée en portant le mélange obtenu à l'étape a), éventuellement après ajout d'eau, à une température inférieure à 20°C, la différence de température entre l'étape a) et l'étape b) étant d'au moins 10°C.

Conformément à un mode de réalisation particulier de l'invention, l'étape b) a lieu sous agitation, de manière à favoriser l'apparition de particules solides de stérols.

Toutefois, l'agitation est de préférence lente, c'est-à-dire de telle sorte que l'apparition de mousse, due à la présence de sels d'acides gras (savons) dans le milieu, soit limitée. L'agitation permet d'homogénéiser la dispersion tout en évitant d'encapsuler les impuretés solubles dans le mélange eau/solvant dans les cristaux en formation.

Par ailleurs, cette étape est de préférence conduite de manière à refroidir lentement la dispersion, dans le but notamment de favoriser une bonne croissance des cristaux, ainsi qu'une pureté appropriée. Par exemple, une baisse de 5 à 10°C par heure est un mode de fonctionnement satisfaisant de l'invention.

L'étape c) du procédé est ensuite effectuée en introduisant les stérols cristallisés, sous forme de dispersion, dans un appareil d'essorage ou de centrifugation. Selon un mode de réalisation préféré, il d'agit d'un appareil d'essorage en rotation. La dispersion est introduite à une vitesse de rotation V₁. Dans un premier temps, on introduit tout ou partie de la dispersion, la quantité introduite étant telle que l'on obtient un gâteau d'épaisseur suffisante et uniforme. Dans un deuxième temps on augmente la vitesse de rotation à une vitesse V₂ supérieure à V₁ mais inférieure à une vitesse de colmatage de l'appareil. L'éventuelle partie restante de la dispersion est introduite lors du deuxième temps.

L'appareillage d'essorage employé peut être tout type d'appareil disponible sur le marché, susceptible de fonctionner à des vitesses variables et répondant aux normes spécifiques d'utilisation de tels appareils.

Les vitesses de rotation, les vitesses de colmatage, et l'épaisseur du gâteau optimales dépendent à la fois de l'appareil et de la composition de la dispersion à essorer. L'homme de l'art, au moyen d'essais de routine, pourra déterminer les conditions d'essorage appropriées.

Généralement, cette opération d'essorage a lieu à une température de l'ordre de 0 à 25°C, de préférence de l'ordre de 5 à 18°C.

Selon une variante de l'invention, la dispersion obtenue à l'étape b) est dilué avant séparation des particules solides, par essorage ou centrifugation. Le diluant employé pour la dilution peut être l'eau, le co-solvant, ou un mélange.

II peut être recommandé de traiter le milieu solvant comprenant les composés saponifiés, à l'issue de cette étape, afin de recycler le co-solvant récupéré, et éventuellement l'eau. Par exemple, on peut acidifier le milieu solvant liquide séparé par centrifugation/essorage récupéré puis mettre en oeuvre toute méthode de purification, comme par exemple la distillation. Ainsi, en tête de la colonne de distillation sera récupéré soit un mélange eau / co-solvant, au cas où un azéotrope existe, soit le co-solvant. En pied de colonne, les composés saponifiés sont récupérés et détruits, ou valorisés.

Une fois l'étape c) de séparation effectuée, on lave éventuellement, lors d'une étape d) le solide obtenu en utilisant un liquide.

Ce qui a été indiqué au sujet de la composition du mélange solvant reste valable pour le liquide de lavage, et ne sera pas repris ici. On peut également utiliser de l'eau sans co-solvant, ou un autre liquide dans lequel les stérols ne sont pas solubilisés, et qui solubilise les composés saponifiés. De préférence, le lavage du solide obtenu est effectué au moyen d'un mélange eau / co-solvant pour lequel le rapport pondéral eau/co-solvant est compris entre 1/20 et 2/1.

En ce qui concerne la quantité mise en oeuvre de liquide par rapport au solide, les quantités sont de préférence telles que le rapport pondéral liquide / solide est compris entre 1/1 et 10/1, de préférence entre 1/1 et 5/1.

Cette étape d) de lavage peut être réalisée de différentes façons, sont dans le même appareil ce que celui utilisé lors de l'étrape, soit un autre appareil, avec des les deux cas éventuellement des opérations de re-dispersion entre l'étape c) et l'étape d).

Selon une première variante, on met en oeuvre l'étape d), on utilise un appareil d'essorage en introduisant dans un appareil d'essorage, à une vitesse de rotation V₁, le liquide, puis on augmente la vitesse de rotation à une vitesse V₂ supérieure à V₁ mais inférieure à une vitesse de colmatage de l'appareil. Dans cette variante, l'étape d) est de préférence mise en oeuvre dans le même appareil que celui de l'étape c).

Selon une deuxième variante, on met en oeuvre l'étape d) en débâtissant le gâteau obtenu à l'issue de l'étape c) de l'appareil d'essorage ou de centrifugation, et en le dispersant dans le liquide, sous agitation, pour obtenir une dispersion. Puis on utilise un appareil d'essorage, et dans un premier temps on introduit dans l'appareil, à une vitesse de rotation V₁, tout ou partie de la dispersion ainsi obtenue de telle sorte que l'on obtient un gâteau d'épaisseur suffisante et uniforme, puis dans un deuxième temps on augmente la vitesse de rotation à une vitesse V₂ supérieure à V₁ mais inférieure à une vitesse de colmatage de l'appareil. L'éventuelle partie restante de la dispersion est introduite lors du deuxième temps.

Cette deuxième variante est préférée dans le cas où un degré élevé de purification des stérols est souhaité.

L'essorage ou la centrifugation peut avoir lieu de manière habituelle à une température proche de la température ambiante (20-25°C). Il en va de même du lavage.

Selon l'étape e) du procédé, l'on reproduit éventuellement au moins une fois l'étape d) précédente. Il est précisé que si plusieurs cycles de lavages sont mis en oeuvre, ils peuvent être réalisés selon l'une et/ou l'autre des deux variantes explicitées ci-dessus.

Les conditions dans lesquelles les séparations et lavages des étapes b) à e) sont effectuées sont de préférence telles que l'on obtienne une teneur en matière sèche d'au moins 60 % en poids, plus particulièrement d'au moins 70 % en poids.

Selon un mode de réalisation avantageux de l'invention, la dernière étape d'essorage ou de lavage réalisée au cours du procédé, en d'autres termes celle de l'étape c) ou celle de l'étape d) si elle est mise en oeuvre, permet d'atteindre une teneur en matière sèche d'au moins 70 % en poids.

Selon le degré de pureté souhaité pour les stérols, il peut être souhaitable de mettre en oeuvre une étape de recristallisation après l'une des étapes c),d) ou e).

Cette étape a lieu de manière classique, par dissolution à chaud des particules solides de stérols dans un solvant et précipitation à froid des particules solides. La composition du solvant est avantageusement celle décrite pour la mise en oeuvre de l'étape c).

Les particules solides obtenues par recristallisation sont séparées du milieu liquide, par exemple par filtration.

Une étape de séchage du solide issu de l'étape c) d) ou e) est ensuite réalisée.

Elle peut avoir lieu selon tout moyen classique. Ainsi, on peut utiliser des méthodes de séchage en étuve, au moyen d'un lit fixe, d'un lit fluide, d'un lit transporté, entre autres.

On ne sortirait pas du cadre de l'invention en mettant en oeuvre des méthodes de séchage sous pression réduite.

De préférence, les températures de séchage mises en oeuvre sont inférieures à 100°C, plus particulièrement comprise entre 40 et 80°C.

Enfin, les conditions de mise en oeuvre de l'opération de séchage (température, pression, durée) sont déterminées de manière à ce que la teneur en matière sèche dans le produit final soit d'au moins 90 % en poids, plus particulièrement d'au moins 95 % en poids, de préférence d'au moins 99% en poids.

On obtient à l'issue du séchage, un produit solide, sous forme de poudre de préférence, comprenant les stérols.

Il est à noter que la pureté en stérols du solide récupéré est d'au moins 95 %. Le rendement est typiquement compris entre 90 et 100 % (quantité de stérols récupérés par rapport à la quantité présente dans le résidu de départ).

La poudre obtenue après le séchage peut éventuellement être mise en forme, selon les méthodes classiques, par exemple sous la forme de granulés, d'écailles ou encore de pastilles ou de perles de diverses géométries.

Les exemples concrets mais non limitatifs de l'invention vont maintenant être détaillés.

### EXEMPLE 1

Dans un réacteur d'un volume utile de 9000 L, en acier Inox 316 L, agité, chauffé par circulation de vapeur dans une double enveloppe et refroidi par circulation d'eau glycolée dans la double enveloppe, on charge les quantités suivantes de potasse et de solvant (eau et éthanol) nécessaires à la saponification de 800 kg de résidu.

| | |
|---|---|
| Résidu | 800 kg |
| Potasse KOH | 138 kg |
| Ethanol | 966 kg |
| Eau | 2151 kg |

Dans cet essai, le rapport pondéral Eau/Ethanol est de 2,23.

Le résidu initial est caractérisé par sa teneur en phytostérols:

| | |
|---|---|
| Indice de saponification | 131,4 mg KOH/g |
| Teneur massique en stérols mesurée par CPG/SM (étalon cholestérol) | 23,23 % |
| Teneur en eau | < 1 % |

L'éthanol pur et l'eau sont introduits dans le réacteur à température ambiante. L'agitation est mise en route (50 tours/min.) et la potasse est introduite dans le réacteur à l'aide d'une trémie à poudres. L'exothermie de solvatation de la potasse dans le solvant éthanolique permet d'augmenter la température du milieu réactionnel. Celui-ci est chauffé à 50°C. Une mesure d'indice de saponification, dite mesure du blanc, est réalisée sur cette solution de potasse éthanolique. Cette mesure du blanc permettra par la suite de quantifier la quantité de potasse consommée par la réaction de saponification. C'est la méthode retenue pour suivre l'avancement de la réaction.
Une fois la potasse introduite et solubilisée dans le solvant composé d'eau et d'éthanol, le résidu est introduit dans le réacteur maintenu à 50°C. Le milieu réactionnel est alors chauffé à la température de reflux total de l'éthanol, environ 80°C. Ces conditions sont maintenues durant 20 heures. Cette durée est estimée suffisante pour que la réaction de saponification soit complète.
A l'issue de la saponfication, le milieu réactionnel, toujours sous agitation, est refroidi de 82°C à 20°C en 510 minutes, soit une cinétique de refroidissement de 0,1°C/min environ. Un phénomène d'agglomération des cristaux en cours de formation est observé sur la paroi du réacteur, sans doute dû à la grande différence de température entre le milieu réactionnel et la surface de la double enveloppe.
La dispersion de cristaux de phytostérols dans un liquide composé d'eau, d'éthanol et de composés saponifiés est ensuite transférée dans une essoreuse à l'aide d'une pompe pneumatique. Cette essoreuse industrielle (modèle ROUSSELET SC 100 Vx) possède un volume utile de 240 L pour une surface filtrante de 1,88 dm². La vitesse d'essorage peut être ajustée entre 100 et 1000 tr/min. L'essoreuse est équipée d'une poche de filtration en polypropylène (débit d'air inférieur à 10 m³/h/m² à 196 Pa).
Le chargement de l'essoreuse se fait en 30 minutes à 750 tr/min. La phase d'essorage proprement dite dure 15 minutes, à une vitesse d'essorage de 1000 tr/min.
On obtient un gateau d'épaisseur uniforme plaqué sur la toile de l'essoreuse. Ce gateau est lavé en place par de l'éthanol à 96%.
Un cycle d'essorage/lavage permet ainsi de récupérer 40 kg environ de gateau lavé, contenant 82% de matière sèche.

Le gâteau obtenu est ensuite séché dans un filtre sécheur de type GUEDU. L'évaporation de l'eau et de l'éthanol est réalisée sous vide à 60°C. 193 kg de phytostérols secs sont récupérés en sortie du sécheur. Leur extrait sec (1h d'étuve à 103 °C) est de 99,76%. Leur pureté est quantifiée par chromatographie en phase gazeuse. Elle est égale à 96 % .On peut ainsi calculer un rendement de récupération des stérols de 0,9976x0,96x193 / (0,2323 x 800) = 97,3%.

### EXEMPLE 2 (comparatif)

### Avec rapport eau / co-solvant inférieur à 1

Dans un réacteur d'un volume utile de 15 Litres, en Inox Uranus, agité à l'aide d'une hélice tripale, chauffé par circulation de vapeur dans une double enveloppe et refroidi par un serpentin alimenté à l'eau de ville dans la cuve, on charge les quantités suivantes de potasse et de solvant nécessaire à la saponification de 3,2 kg de résidu.

| | |
|---|---|
| Résidu | 3200 |
| Potasse KOH | 2746 |
| Ethanol | 6144 |
| Eau | 630 |
| Eau de relargage | 5000 |

Dans cet essai, le rapport pondéral Eau/Ethanol est de 0,92.
Caractéristiques du résidu :

| | |
|---|---|
| Indice de saponification | 115 mg KOH/g |
| Teneur massique en stérols mesurée par CPG/SM (étalon cholestérol) | 23,62 % |
| Teneur en eau | < 1 % |

L'éthanol pur et l'eau sont introduits dans le réacteur à température ambiante. L'agitation est mise en route (250 tours/min.) et la potasse est introduite dans le réacteur à l'aide d'une trémie à poudres. L'exothermie de solvatation de la potasse dans le solvant éthanolique permet d'augmenter la température du milieu réactionnel. Une mesure d'indice de saponification est réalisée sur cette solution de potasse alcoolique. Cette mesure de blanc permettra par la suite de quantifier la quantité de potasse consommée par la réaction de saponification. C'est la méthode retenue pour suivre l'avancement de la réaction.

Une fois la potasse introduite et solubilisée dans le mélange de solvants eau et éthanol, le résidu est introduit dans le réacteur. Le milieu réactionnel est alors chauffé à la température de reflux total de l'éthanol, environ 80°C. Ces conditions sont maintenues jusqu'à ce que l'indice de saponification du milieu réactionnel atteigne un seuil maximum constant, indiquant ainsi que la réaction de saponification ne consomme plus de potasse. Ce maximum est atteint après 40 minutes de maintien des conditions opératoires (température de reflux total d'éthanol et agitation à 250 tr/min). Après 65 minutes de réaction, la quantité d'eau de relargage, préalablement chauffée à la température du milieu réactionnel (78°C), est introduite dans le réacteur. Le milieu réactionnel, maintenu sous agitation, est refroidi de 78°C à 25°C en 105 minutes, soit une cinétique de refroidissement de 0.5°C/min.
Une dispersion de cristaux de phytostérols dans un liquide composé d'eau, d'éthanol et de composés saponifiés est ainsi obtenu.
Cette dispersion est filtrée sur un Büchner de surface utile 0,56 m² équipé d'une toile de polypropylène (perméabilité à l'air de 75 m³/h/m² à 196 Pa), sous vide absolu de 100 mbar obtenu par une pompe à anneau liquide
Cette opération de filtration est rendue difficile par la présence d'une couche solide homogène visqueuse qui colmate le filtre. Le lavage des cristaux est rendu impossible. Une étape supplémentaire de recristallisation dans l'éthanol a ainsi dû être ajoutée au procédé. Les cristaux ainsi recristallisés sont faciles à filtrer et peuvent être lavés à l'eau, en place sur le Büchner.
Les cristaux recueillis sont séchés dans une étuve, puis pesés. 420 g sont obtenus. Leur pureté est quantifié par chromatographie en phase gazeuse. Elle est égale à 100% On peut ainsi calculer un rendement de récupération des stérols de 420 / (0,2362 x 3200) = 55,6 %.

## Revendications

1. Utilisation d'un produit issu du traitement d'huiles de composés d'origine végétale, de préférence oléagineuse, pour la récupération de stérols, **caractérisé en ce que** le produit utilisé est un résidu lourd issu d'une distillation d'un produit de transestérification de l'huile, ledit résidu comprenant :
- entre 15% et 50 % en poids de stérols,
- et des composés saponifiables.

2. Utilisation selon la revendication 1, **caractérisée en ce que** le résidu comprend entre 15 et 30 % en poids de stérols.

3. Utilisation selon l'une des revendications précédentes, **caractérisée en ce que** l'indice de saponification du résidu est compris entre 100 et 150 grammes de potasse par gramme de résidu.

4. Utilisation selon l'une des revendications précédentes, **caractérisée en ce que** l'huile est l'huile de colza, l'huile de tournesol, l'huile d'arachide, l'huile d'olive, l'huile de noix, l'huile de maïs, l'huile de soja, l'huile d'avocat, l'huile de lin, l'huile de chanvre, l'huile de noix de macadamia, l'huile de pépins de raisin, l'huile de coprah, l'huile de palme, l'huile de graines de coton, l'huile de sésame, l'huile de ricin, l'huile de babassu, l'huile de palmiste, l'huile de tung, ou le beurre de karité, seule ou en mélange.

5. Procédé de récupération de stérols à partir d'un produit issu du traitement d'huiles de composés d'origine végétale oléagineuse, **caractérisé en ce que**:
- le produit utilisé est un résidu lourd issu d'une distillation d'un produit de transestérification de l'huile, ledit résidu comprenant entre 15% et 50 % en poids de stérols, et des composés saponifiables, et
- le procédé comprend les étapes suivantes:
a) on saponifie les composés saponifiables par mise en contact du résidu avec une base en présence d'un solvant comprenant de l'eau et un co-solvant,
b) on cristallise les stérols sous la forme de particules solides en présence du mélange solvant à un rapport pondéral eau / co-solvant supérieur à 1/1, le cas échéant en ajoutant de l'eau de manière à atteindre ce rapport pondéral, de manière à obtenir une dispersion,
c) on sépare les stérols cristallisés du mélange solvant issu de l'étape b), comprenant des composés saponifiés, par essorage ou centrifugation de la dispersion,
d) on lave éventuellement les stérols avec un liquide et l'on élimine ledit liquide par essorage ou centrifugation,
e) on reproduit éventuellement au moins une fois l'étape d),
f) on sèche et récupère les stérols.

6. Procédé selon la revendication 5, **caractérisé en ce que** le résidu comprend entre 15 et 30 % en poids de stérols.

7. Procédé selon l'une des revendications 5 ou 6, **caractérisé en ce que** l'indice de saponification du résidu est compris entre 100 et 150 grammes de potasse par gramme de résidu.

8. Procédé selon l'une des revendications 5 à 7, **caractérisée en ce que** l'huile est l'huile de colza, l'huile de tournesol, l'huile d'arachide, l'huile d'olive, l'huile de noix, l'huile de maïs, l'huile de soja, l'huile d'avocat, l'huile de lin, l'huile de chanvre, l'huile de noix de macadamia, l'huile de pépins de raisin, l'huile de coprah, l'huile de palme, l'huile de graines de coton, l'huile de sésame, l'huile de ricin, l'huile de babassu, l'huile de palmiste, l'huile de tung, ou le beurre de karité, seule ou en mélange.

9. Procédé selon l'une des revendications 5 à 8, **caractérisé en ce que** l'on met en oeuvre l'étape a) en présence d'une base choisie parmi les hydroxydes de métal alcalin ou alcalino-terreux, et de préférence parmi la potasse, la soude.

10. Procédé selon l'une des revendications 5 à 9, **caractérisé en ce que** l'on ajoute une quantité de base supérieure ou égale à la quantité nécessaire pour saponifier la totalité des composés saponifiables présents dans le résidu.

11. Procédé selon l'une des revendications 5 à 10, **caractérisé en ce que** le mélange solvant et le liquide solubilisent les composés saponifiés et ne solubilisent pas les stérols.

12. Procédé selon la revendication précédente, **caractérisé en ce que** le co-solvant dans le mélange solvant est partiellement ou totalement soluble dans l'eau, la teneur en co-solvant dans le mélange solvant ne dépassant pas sa limite de solubilité dans l'eau.

13. Procédé selon l'une des revendications 11 ou 12, **caractérisé en ce que** le co-solvant est choisi parmi les alcools saturés comprenant 1 à 4 atomes de carbone.

14. Procédé selon l'une des revendications 11 à 13, **caractérisé en ce que** le rapport pondéral eau / co-solvant du mélange utilisé à l'étape a) est supérieur à 1/1 et inférieur ou égal à 3/1, de préférence compris entre 1,5/1 et 3/1, et plus préférentiellement compris entre 2/1 et 3/1.

15. Procédé selon l'une des revendications 5 à 14, **caractérisé en ce que** pour l'étape a) le rapport pondéral mélange solvant / résidu est compris entre 2/1 et 10/1, de préférence entre 2/1 et 5/1.

16. Procédé selon l'une des revendications 5 à 15, **caractérisé en ce que** l'on met en oeuvre l'étape a) à une température supérieure à 20°C et de préférence à la température de reflux du co-solvant.

17. Procédé selon l'une des revendications 5 à 16, **caractérisé en ce que** lors de l'étape b) la température est inférieure à 20°C, la différence de température entre l'étape a) et l'étape b) étant d'au moins 10°C.

18. Procédé selon l'une des revendications 5 à 17, **caractérisé en ce que** l'étape c) est mise en oeuvre avec un appareil d'essorage en rotation, dans un premier temps en introduisant la dispersion à une vitesse de rotation V₁, en quantité telle que l'on obtient un gâteau d'épaisseur suffisante et uniforme, puis dans un deuxième temps en augmentant la vitesse de rotation à une vitesse V₂ supérieure à V₁ mais inférieure à une vitesse de colmatage de l'appareil.

19. Procédé selon l'une des revendications 5 à 18, **caractérisé en ce que** l'étape d) est mise en oeuvre avec un appareil d'essorage en rotation, dans un premier temps en introduisant le liquide à une vitesse de rotation V₁, puis dans un deuxième temps en augmentant la vitesse de rotation à une vitesse V₂ supérieure à V₁ mais inférieure à une vitesse de colmatage de l'appareil.

20. Procédé selon l'une des revendications 5 à 18, **caractérisé en ce que** l'on met en oeuvre l'étape d) en extrayant le gâteau obtenu à l'issue de l'étape c) d'un appareil d'essorage ou de centrifugation, et en le dispersant dans le liquide, sous agitation, de manière à obtenir une dispersion,; puis on introduit dans l'appareil d'essorage ou de centrifugation, à une vitesse de rotation V₁, la dispersion ainsi obtenue de telle sorte que l'on obtient un gâteau d'épaisseur suffisante et uniforme, puis on augmente la vitesse de rotation à une vitesse V₂ supérieure à V₁ mais inférieure à la vitesse de colmatage de l'appareil.

21. Procédé selon l'une des revendications 19 ou 20, **caractérisé en ce que** le rapport pondéral liquide / gâteau ou liquide / produit cristallisé est compris entre 1/1 et 10/1, de préférence entre 1/1 et 5/1.

22. Procédé selon l'une des revendications 5 à 21, **caractérisé en ce que** l'on effectue l'essorage ou la centrifugation jusqu'à obtenir une teneur en matière sèche d'au moins 60 % en poids, plus particulièrement d'au moins 70 % en poids.

23. Procédé selon l'une des revendications 5 à 22, **caractérisé en ce que** l'on effectue l'étape f) dans des conditions de température et de pression telles que la teneur en matière sèche est d'au moins 90 % en poids, plus particulièrement d'au moins 95 % en poids, de préférence d'au moins 99% en poids.

## Claims

1. Use of a product stemming from the treatment of oils of compounds originating from plants, preferably from oleaginous plants, for recovering sterols, **characterized in that** the product used is a heavy residue from distillation of a transesterification product of the oil, said residue comprising:
- between 15% en 50% by weight of sterols,
- and saponifiable compounds.

2. The use according to claim 1, **characterized in that** the residue comprises between 15 and 30% by weight of sterols.

3. The use according to one of the preceding claims, **characterized in that** the saponification index of the residue is comprised between 100 and 150 grams of potash per gram of residue.

4. The use according to one of the preceding claims, **characterized in that** the oil is rapeseed oil, sunflower oil, groundnut oil, olive oil, walnut oil, corn oil, soya bean oil, avocado oil, linseed oil, hempseed oil, macadamia nut oil, grapeseed oil, coconut oil, palm oil, cottonseed oil, sesame oil, castor oil, babassu oil, palm kernel oil, tung oil, or shea butter, either alone or as a mixture.

5. A method for recovering sterols from a product stemming from the treatment of oils of compounds originating from oleaginous plants, **characterized in that**:
- the product used is a heavy residue from distillation of a transesterification product of oil, said residue comprising between 15% and 50% by weight of sterols, and saponifiable compounds, and
- the method comprising the following steps:
a) the saponifiable compounds are saponified by putting the residue in contact with a base in the presence of a solvent comprising water and a co-solvent,
b) the sterols are crystallized as solid particles in the presence of the solvent mixture at a water/co-solvent weight ratio larger than 1:1, if necessary by adding water in order to reach this weight ratio, so as to obtain a dispersion,
c) the crystallized sterols of the solvent mixture from step b) comprising saponified compounds, are separated by spin-drying or centrifugation of the dispersion,
d) the sterols are optionally washed with a liquid and said liquid is removed by spin-drying or centrifugation,
e) step d) is optionally reproduced at least once,
f) the sterols are dried and recovered.

6. The method according to claim 5, **characterized in that** the residue comprises between 15 and 30% by weight of sterols.

7. The method according to one of claims 5 or 6, **characterized in that** the saponification index of the residue is comprised between 100 and 150 grams of potash per gram of residue.

8. The method according to one of claims 5 to 7, **characterized in that** the oil is rapeseed oil, sunflower oil, groundnut oil, olive oil, walnut oil, corn oil, soya bean oil, avocado oil, linseed oil, hempseed oil, macadamia nut oil, grapeseed oil, coconut oil, palm oil, cottonseed oil, sesame oil, castor oil, babassu oil, palm kernel oil, tung oil, or shea butter, either alone or as a mixture.

9. The method according to one of claims 5 to 8, **characterized in that** step a) is carried out in the presence of a base selected from alkaline or alkaline earth metal hydroxides and preferably from potash, soda.

10. The method according to one of claims 5 to 9, **characterized in that** an amount of base is added, larger than or equal to the amount required for saponifying the totality of the saponifiable compounds present in the residue.

11. The method according to one of claims 5 to 10, **characterized in that** the solvent mixture and the liquid solubilize the saponified compounds and do not solubilize the sterols.

12. The method according to the preceding claim, **characterized in that** the co-solvent in the solvent mixture is partially or totally soluble in water, the co-solvent content in the solvent mixture not exceeding its solubility limit in water.

13. The method according to one of claims 11 or 12, **characterized in that** the co-solvent is selected from saturated alcohols comprising 1 to 4 carbon atoms.

14. The method according to one of claims 11 to 13, **characterized in that** the water/co-solvent weight ratio of the mixture used in step a) is larger than 1:1 and less than 3:1, preferably comprised between 1.5:1 and 3:1, and more preferentially comprised between 2:1 and 3:1.

15. The method according to one of claims 5 to 14, **characterized in that** for step a) the solvent/residue mixture weight ratio is comprised between 2:1 and 10:1, preferably between 2:1 and 5:1.

16. The method according to one of claims 5 to 15, **characterized in that** step a) is carried out at a temperature above 20°C and preferably at the reflux temperature of the co-solvent.

17. The method according to one of claims 5 to 16, **characterized in that**, during step b), the temperature is less than 20°C, the temperature difference between step a) and step b) being at least 10°C.

18. The method according to one of claims 5 to 17, **characterized in that** step c) is carried out with a rotating spin-drying apparatus, in a first time while introducing the dispersion at a rotational speed V₁, in such an amount that a cake with sufficient and uniform thickness is obtained, and then in a second time while increasing the rotational speed to a speed V₂ greater than V₁, but less than a speed at which there is clogging of the apparatus.

19. The method according to one of claims 5 to 18, **characterized in that** step d) is carried out with a rotational spin-drying apparatus, in a first time while introducing the liquid at a rotational speed V₁, and then in a second phase by increasing the rotational speed to a speed V₂ greater than V₁, but less than a speed at which there is clogging of the apparatus.

20. The method according to one of claims 5 to 18, **characterized in that** step d) is carried out while extracting the cake obtained upon completion of step c) from a spin-drying or centrifugation apparatus, and while dispersing it in the liquid, under stirring, so as to obtain a dispersion; and the thereby obtained dispersion is then introduced into the spin-drying or centrifugation apparatus, at a rotational speed V₁, so as to obtain a cake with sufficient and uniform thickness, and then the rotational speed is increased to a speed V₂ greater than V₁, but less than the speed at which there is clogging of the apparatus.

21. The method according to one of claims 19 or 20, **characterized in that** the liquid/cake or liquid/crystallized product weight ratio is comprised between 1:1 and 10:1, preferably between 1:1 and 5:1.

22. The method according to one of claims 5 to 21, **characterized in that** spin-drying or centrifugation is carried out until a dry material content of at least 60% by weight, more particularly of at least 70% by weight, is obtained.

23. The method according to one of claims 5 to 22, **characterized in that** step f) is carried out under temperature and pressure conditions such that the dry material content is at least 90% by weight, more particularly at least 95% by weight, preferably at least 99% by weight.

## Patentansprüche

1. Verwendung eines Produkts aus der Behandlung von Ölen von Verbindungen pflanzlichen, vorzugsweise ölpflanzlichen Ursprungs zur Gewinnung von Sterolen, **dadurch gekennzeichnet, daß** das verwendete Produkt ein schwerer Rückstand aus einer Destillation eines Transesterifizierungsprodukts des Öls ist, wobei der Rückstand umfaßt:
- zwischen 15 und 50 Gewichtsprozent Sterole
- und verseifbare Verbindungen.

2. Verwendung nach Anspruch 1, **dadurch gekennzeichnet, daß** der Rückstand zwischen 15 und 30 Gewichtsprozent Sterole umfaßt.

3. Verwendung nach einem der vorhergehenden Ansprüche, **dadurch gekennzeichnet, daß** der Verseifungsindex des Rückstandes zwischen 100 und 150 Gramm Kaliumhydroxid pro Gramm Rückstand liegt.

4. Verwendung nach einem der vorhergehenden Ansprüche, **dadurch gekennzeichnet, daß** das Öl Rapsöl, Sonnenblumenöl, Erdnußöl, Olivenöl, Nußöl, Maisöl, Sojaöl, Avocadoöl, Leinöl, Hanföl, Macadamianußöl, Traubenkernöl, Coprahöl, Palmöl, Baumwollsamenöl, Sesamöl, Ricinusöl, Babassuöl, Palmkernöl, Tungöl oder Sheabutter, allein oder im Gemisch ist.

5. Verfahren zur Gewinnung von Sterolen aus einem Produkt aus der Behandlung von Ölen von Verbindungen ölpflanzlichen Ursprungs, **dadurch gekennzeichnet, daß**
- das verwendete Produkt ein schwerer Rückstand aus einer Destillation eines Transesterifizierungsproduktes des Öls ist, wobei der Rückstand zwischen 15 und 50 Gewichtsprozent Sterole und verseifbare Verbindungen umfaßt, und
- das Verfahren die folgenden Schritte umfaßt:
a) man verseift die verseifbaren Verbindungen durch Kontaktieren des Rückstandes mit einer Base in Gegenwart eines Lösungsmittels, das Wasser und ein Co-Lösungsmittel umfaßt,
b) man kristallisiert die Sterole in Form von festen Partikeln in Gegenwart des Lösungsmittelgemischs bei einem Gewichtsverhältnis Wasser/Co-Lösungsmittel größer als 1/1, gegebenenfalls unter Zugabe von Wasser derart, daß dieses Gewichtsverhältnis erreicht wird, derart, daß eine Dispersion erhalten wird,
c) man trennt die kristallisierten Sterole von dem Lösungsmittelgemisch aus Schritt b), umfassend verseifte Verbindungen, durch Ausschleudern oder Zentrifugieren der Dispersion,
d) man wäscht gegebenenfalls die Sterole mit einer Flüssigkeit und man eliminiert die Flüssigkeit durch Ausschleudern oder Zentrifugieren,
e) man reproduziert gegebenenfalls wenigstens einmal Schritt d),
f) man trocknet und gewinnt die Sterole.

6. Verfahren nach Anspruch 5, **dadurch gekennzeichnet, daß** der Rückstand 15 bis 30 Gewichtsprozent Sterole umfaßt.

7. Verfahren nach einem der Ansprüche 5 oder 6, **dadurch gekennzeichnet, daß** der Verseifungsindex des Rückstandes zwischen 100 und 150 Gramm Kaliumhydroxid pro Gramm Rückstand liegt.

8. Verfahren nach einem der Ansprüche 5 bis 7, **dadurch gekennzeichnet, daß** das Öl Rapsöl, Sonnenblumenöl, Erdnußöl, Olivenöl, Nußöl, Maisöl, Sojaöl, Avocadoöl, Leinöl, Hanföl, Macadamianußöl, Traubenkernöl, Coprahöl, Palmöl, Baumwollsamenöl, Sesamöl, Ricinusöl, Babassuöl, Palmkernöl, Tungöl oder Sheabutter, allein oder im Gemisch ist.

9. Verfahren nach einem der Ansprüche 5 bis 8, **dadurch gekennzeichnet, daß** man Schritt a) in Gegenwart einer Base ausführt, gewählt unter den Alkali- oder Erdalkalimetallhydroxiden und vorzugsweise unter Kaliumhydroxid, Natriumhydroxid.

10. Verfahren nach einem der Ansprüche 5 bis 9, **dadurch gekennzeichnet, daß** man eine Basemenge größer oder gleich der Menge zugibt, die notwendig ist, um die Gesamtheit der verseifbaren Verbindungen zu verseifen, die in dem Rückstand vorliegen.

11. Verfahren nach einem der Ansprüche 5 bis 10, **dadurch gekennzeichnet, daß** das Lösungsmittelgemisch und die Flüssigkeit die verseiften Verbindungen lösen und die Sterole nicht lösen.

12. Verfahren nach dem vorhergehenden Anspruch, **dadurch gekennzeichnet, daß** das Co-Lösungsmittel in dem Lösungsmittelgemisch teilweise oder vollständig in Wasser löslich ist, wobei der Gehalt an Co-Lösungsmittel in dem Lösungsmittelgemisch nicht die Löslichkeitsgrenze in Wasser überschreitet.

13. Verfahren nach einem der Ansprüche 11 oder 12, **dadurch gekennzeichnet, daß** das Co-Lösungsmittel gewählt ist unter den gesättigten Alkoholen, die 1 bis 4 Kohlenstoffatome umfassen.

14. Verfahren nach einem der Ansprüche 11 bis 13, **dadurch gekennzeichnet, daß** das Gewichtsverhältnis Wasser/Co-Lösungsmittel des in Schritt a) verwendeten Gemisches größer als 1/1 und kleiner oder gleich 3/1 ist, vorzugsweise zwischen 1,5/1 und 3/1 liegt und bevorzugter zwischen 2/1 und 3/1 liegt.

15. Verfahren nach einem der Ansprüche 5 bis 14, **dadurch gekennzeichnet, daß** für Schritt a) das Gewichtsverhältnis Lösungsmittelgemisch/Rückstand zwischen 2/1 und 10/1, vorzugsweise zwischen 2/1 und 5/1 liegt.

16. Verfahren nach einem der Ansprüche 5 bis 15, **dadurch gekennzeichnet, daß** man Schritt a) bei einer Temperatur über 20°C und vorzugsweise bei der Rückflußtemperatur des Co-Lösungsmittels ausführt.

17. Verfahren nach einem der Ansprüche 5 bis 16, **dadurch gekennzeichnet, daß** bei Schritt b) die Temperatur kleiner als 20°C ist, wobei die Temperaturdifferenz zwischen Schritt a) und Schritt b) wenigstens 10°C ist.

18. Verfahren nach einem der Ansprüche 5 bis 17, **dadurch gekennzeichnet, daß** Schritt c) ausgeführt wird mit einer Rotierschleudervorrichtung, zu einem ersten Zeitpunkt unter Einführen der Dispersion bei einer Rotationsgeschwindigkeit V₁ in einer Menge derart, daß man einen Kuchen von ausreichender und gleichförmiger Dicke erhält, und dann zu einem zweiten Zeitpunkt unter Erhöhung der Rotationsgeschwindigkeit auf eine Geschwindigkeit V₂ größer als V₁ aber unter der Verstopfungsgeschwindigkeit der Vorrichtung.

19. Verfahren nach einem der Ansprüche 5 bis 18, **dadurch gekennzeichnet, daß** der Schritt d) ausgeführt wird mit einer Rotationsschleudervorrichtung zu einem ersten Zeitpunkt unter Einführen der Flüssigkeit bei einer Rotationsgeschwindigkeit V₁ und dann zu einem zweiten Zeitpunkt unter Erhöhung der Rotationsgeschwindigkeit auf eine Geschwindigkeit V₂ größer als V₁ aber unter der Verstopfungsgeschwindigkeit der Vorrichtung.

20. Verfahren nach einem der Ansprüche 5 bis 18, **dadurch gekennzeichnet, daß** man Schritt d) ausführt unter Extrahieren des Kuchens, der erhalten ist aus Schritt c), mit einer Schleuder- oder Zentrifugiervorrichtung und indem man ihn in der Flüssigkeit dispergiert unter Rühren derart, daß eine Dispersion erhalten wird, und man dann in die Schleuder- oder Zentrifugiervorrichtung bei einer Rotationsgeschwindigkeit V₁ die so erhaltene Dispersion derart einführt, daß man einen Kuchen von ausreichender und gleichförmiger Dicke erhält, und man dann die Rotationsgeschwindigkeit auf eine Geschwindigkeit V₂ größer als V₁ aber unter der Verstopfungsgeschwindigkeit der Vorrichtung erhöht.

21. Verfahren nach einem der Ansprüche 19 oder 20, **dadurch gekennzeichnet, daß** das Gewichtsverhältnis Flüssigkeit/Kuchen oder Flüssigkeit/kristallisiertes Produkt zwischen 1/1 und 10/1, vorzugsweise zwischen 1/1 und 5/1 liegt.

22. Verfahren nach einem der Ansprüche 5 bis 21, **dadurch gekennzeichnet, daß** man das Ausschleudern oder das Zentrifugieren ausführt bis zum Erhalt eines Gehalts an Trockenmaterial von wenigstens 60 Gewichtsprozent, spezieller wenigstens 70 Gewichtsprozent.

23. Verfahren nach einem der Ansprüche 5 bis 22, **dadurch gekennzeichnet, daß** man Schritt f) unter Temperatur- und Druckbedingungen derart ausführt, daß der Gehalt an Trockenmaterial wenigstens 90 Gewichtsprozent, spezieller wenigstens 95 Gewichtsprozent, vorzugsweise wenigstens 99 Gewichtsprozent ist.
